# EUROPÄISCHE PATENTSCHRIFT

(11) **EP 3 509 521 B1**
(45) Veröffentlichungstag und Bekanntmachung des Hinweises auf die Patenterteilung: **04.11.2020**
(21) Anmeldenummer: 17777488.2
(22) Anmeldetag: 07.09.2017
(51) Int. Cl.: A61B 18/14, A61N 7/02, A61N 1/05, A61B 18/00, A61N 7/00

(54) **VORRICHTUNG ZUR VORBEUGUNG UND BEHANDLUNG EINES VASOSPASMUS**
DEVICE FOR PREVENTING AND TREATING A VASOSPASM
DISPOSITIF POUR LA PRÉVENTION ET LE TRAITEMENT D'UN VASOSPASME

(30) Priorität: 08.09.2016 DE 102016116871
(43) Veröffentlichungstag der Anmeldung: 17.07.2019
(73) Patentinhaber: Phenox GmbH, 44801 Bochum (DE)
(72) Erfinder: HENKES, Hans, 70192 Stuttgart (DE); HANNES, Ralf, 44137 Dortmund (DE); MONSTADT, Hermann, 44797 Bochum (DE)
(74) Vertreter: Schneiders & Behrendt PartmbB Rechtsanwälte - Patentanwälte
(86) Internationale Anmeldenummer: PCT/EP2017/072451
(87) Internationale Veröffentlichungsnummer: WO 2018/046592

(56) Entgegenhaltungen:
- EP-A1- 2 732 784
- DE-A1-102011 053 021
- DE-A1-102014 101 348
- US-A1- 2007 129 760
- US-A1- 2015 018 818

## Beschreibung

Die Erfindung betrifft eine Vorrichtung mit einer Stentstruktur, die zur Einbringung in intrakranielle Blutgefäße des menschlichen oder tierischen Körpers vorgesehen ist, wobei die Stentstruktur einen expandierten Zustand, in dem sie in der Lage ist, sich an die Innenwandung des Blutgefäßes anzulegen, und einen komprimierten Zustand aufweist, in dem sie innerhalb eines Mikrokatheters durch das Blutgefäß bewegbar ist, wobei die Stentstruktur mit einer Einführhilfe verbunden ist und wobei die Stentstruktur in der Lage ist, nach Freisetzung aus dem Mikrokatheter selbständig in den expandierten Zustand überzugehen. Die Vorrichtung dient der Vorbeugung bzw. Behandlung eines Vasospasmus.

Unter einem Vasospasmus versteht man eine krampfartige Verengung eines Blutgefäßes. Hiermit ist die Gefahr verbunden, dass nachfolgende Gefäße nicht mehr in ausreichendem Maße mit Blut versorgt werden (Ischämie), was zur Nekrose des durch die Gefäße mit Blut versorgten Gewebes führen kann. Gerade im zerebralen Bereich kann ein Vasospasmus einige Tage nach einer Subarachnoidalblutung (SAB) auftreten, häufig als Folge der Ruptur eines Aneurysmas. Weitere Ursachen von Subarachnoidalblutungen sind Schädel-Hirn-Traumata und Blutungen aus Gefäßmißbildungen oder Tumoren. Ausgetretenes Blut im Subarachnoidalraum umspült die dort verlaufenden Gefäße und gilt als der wichtigste auslösende Faktor des Vasospasmus. Etwa 60% aller SAB-Patienten erfahren etwa zwischen dem fünften und zwanzigsten Tag nach der Blutung einen mehr oder weniger stark ausgeprägten Vasospasmus. Bei hochgradiger Einengung der arteriellen Gefäße kommt es zu einer Unterversorgung des abhängigen Hirngewebes, das dadurch irreversiblen Schaden erleiden kann (Hirninfarkt). Etwa 15-20% aller Patienten die primär eine SAB überlebt haben, erfahren eine dauerhafte neurologische Schädigung mit daraus resultierender Behinderung. Etwa 5% der primär überlebenden SAB-Patienten stirbt im weiteren Verlauf an den Folgen von zerebralem Vasospasmus. Insofern ist ein Vasospasmus einer der Hauptgründe für nach einer Ruptur eines Aneurysmas und/oder Blutung aus demselben oder einer Operation in diesem Bereich auftretende Schlaganfälle bis hin zu Todesfällen.

Üblicherweise wird ein Vasospasmus medikamentös behandelt, wobei insbesondere Calciumkanalblocker oder Medikamente zum Einsatz kommen, die das NO-Level im Blut erhöhen. Ein Beispiel für einen Calciumkanalblocker ist Nimodipin, welches häufig nach Subarachnoidalblutungen eingesetzt wird, um Vasospasmen vorzubeugen. Die medikamentöse Behandlung ist jedoch mit nicht unerheblichen Nebenwirkungen behaftet und darüber hinaus kosten- und zeitintensiv.

Weitere Möglichkeiten zur Behandlung eines Vasospasmus sind intensivmedizinische Maßnahmen wie die Anhebung des arteriellen Blutdrucks und die Erhöhung des zirkulierenden Blutvolumens, die Erweiterung verengter Gefäße mit Hilfe eines Ballons, die Blockade des Ganglion stellatum und die operative Zerstörung sympathischer Nervenfasern (Sympathikolyse). Diese Behandlungsmethoden sind in ihrer Wirksamkeit individuell uneinheitlich, z. T. sehr aufwändig, und häufig nicht ausreichend lange anhaltend wirksam. Die Blockade des Ganglion stellatum und die operative Sympathikolyse sind zwar wirksam, weil die sympathischen Nervenfasern in der Wand der Hirnarterien wesentlich an der Entstehung des zerebralen Vasospasmus beteiligt sind. Zur vollumfänglichen Vermeidung und Behandlung eines zerebralen Vasospasmus sind die Verfahren jedoch unzureichend, da die Blockade des Ganglion stellatums nur wenige Stunden anhält und eine operative Sympathektomie nur auf ein eng umschriebenes Gefäßsegment beschränkt bleibt, das zu diesem Zweck operativ präpariert werden muss.

DE 10 2014 101 348 A1 beschreibt eine Vorrichtung zur Ablation von Gewebezellen, US 2015/0018818 A1 beschreibt eine Vorrichtung zur Nervenmodulation oder -ablation. DE 10 2011 053 021 A1 befasst sich mit einer Vorrichtung zur Neuromodulation und/oder Nervenstimulation und EP 2 732 784 A1 mit der Applikation von Hochfrequenzenergie an der Renal-Arterienwand. US 2007/129760 A1 betrifft eine Vorrichtung zur intravaskulären Neuromodulation.

Es stellte sich somit die Aufgabe, Mittel zur Verfügung zu stellen, die die Vorbeugung und Behandlung eines Vasospasmus in anderer Art und Weise erlauben.

Diese Aufgabe wird erfindungsgemäß gelöst durch eine Vorrichtung gemäß Anspruch 1. Die abhängigen Ansprüche definieren bevorzugte Ausgestaltungen der Erfindung.

Aspekte, Ausführungsformen, Ausgestaltungen und Beispiele, die nicht unter den Wortlaut der Ansprüche fallen, sind nicht Teil der Erfindung. Nachstehend beschriebene Verfahren sind ebenfalls nicht Teil der Erfindung.

Diese Offenbarung beschreibt eine Vorrichtung mit einer Stentstruktur, die zur Einbringung in intrakranielle Blutgefäße des menschlichen oder tierischen Körpers vorgesehen ist, wobei die Stentstruktur einen expandierten Zustand, in dem sie in der Lage ist, sich an die Innenwandung des Blutgefäßes anzulegen, und einen komprimierten Zustand aufweist, in dem sie innerhalb eines Mikrokatheters durch das Blutgefäß bewegbar ist, wobei die Stentstruktur mit einer Einführhilfe verbunden ist und wobei die Stentstruktur in der Lage ist, nach Freisetzung aus dem Mikrokatheter selbständig in den expandierten Zustand überzugehen, wobei die Stentstruktur elektrische Leiter aufweist, über die elektrische Impulse, Hochfrequenzimpulse oder Ultraschallimpulse auf in der Gefäßwand des Blutgefäßes verlaufende Nervenfasern applizierbar sind, um die Funktion der Nervenfasern vorübergehend oder dauerhaft zu mindern und einem Vasospasmus vorzubeugen oder diesen zu behandeln.

Die Erfindung beruht somit darauf, eine Stentstruktur zur endovaskulären Denervation hirnversorgender Arterien einzusetzen. Endovaskuläre Verfahren zur Denervation sympathischer Nervenfasern sind zwar im Bereich der Denervation der Arteria renalis bekannt, diese dient jedoch der Unterbrechung von Nervenfasern zwischen Gehirn und Niere, um die Freisetzung von blutdruckerhöhenden Stoffen zu reduzieren. Darüber hinaus sind hierfür verwendete Ballonkatheter nicht für den intrakraniellen Bereich geeignet.

Physikalisch kann die Applikation von Impulsen auf die Nervenfasern in Form von Hochfrequenz (HF)-Signalen, Gleichstrom, Wechselstrom oder Ultraschall erfolgen. In der Regel beruht die Denervation letztlich auf einer Erwärmung der Gefäßwand, die zu einer Ausschaltung oder Minderung der Funktion der Nervenfasern führt. Die Anwendung von Hochfrequenz- oder Ultraschallimpulsen ist insofern bevorzugt, als hierbei Energiemaxima in der Tiefe der umgebenden Gefäßwand erzeugt werden können, sodass gezielt die Nervenfasern, nicht hingegen die gesamte Gefäßwand geschädigt wird. Bei den Nervenfasern handelt es sich um solche des sympathischen Nervensystems.

Eine einzelne Impulsapplikation auf die Nervenfasern währt typischerweise über eine Zeit von 30 bis 120 sec, wobei die Nervenfasern auf 50 bis 80°C erwärmt werden können. Die Eindringtiefe der Energie in die Gefäßwand beträgt beispielsweise 1 bis 3 mm. Im Falle von HF-Impulsen liegt die Frequenz typischerweise bei 300 bis 4.000 kHz. Unter Hochfrequenz im Sinne dieser Erfindung werden elektromagnetische Wellen mit einer Frequenz > 1 kHz verstanden, einschließlich Mikrowellen mit einer Frequenz > 1 GHz.

Stents, auch Gefäßendoprothesen genannt, werden häufig zur Behandlung von Gefäßverengungen eingesetzt und an der Stelle der Gefäßverengung dauerhaft implantiert, um das Gefäß offen zu halten. Typischerweise weisen Stents eine Röhrenstruktur auf und sind entweder lasergeschnitten, so dass sich eine Oberfläche aus Streben ergibt, zwischen denen Öffnungen vorliegen, oder bestehen aus einem Drahtgeflecht. Stents können durch einen Katheter an den Zielort gebracht und dort expandiert werden; im Falle selbstexpandierender Stents aus Formgedächtnismaterialien erfolgt diese Expansion und die Anlegung an die Gefäßinnenwand selbständig. Nach endgültiger Platzierung verbleibt nur der Stent selbst am Zielort; Katheter, Führungsdrähte und andere Hilfsmittel werden aus dem Blutgefäßsystem entfernt. Ähnlich aufgebaute Implantate mit höherer Oberflächendichte, sog. Flow Diverter, werden auch zum Verschließen von Aneurysmen eingesetzt, indem sie vor dem Hals eines Aneurysmas platziert werden. Die Vorbeugung oder Behandlung eines Vasospasmus mit Hilfe einer Stentstruktur wurde hingegen bislang noch nicht beschrieben.

Die erfindungsgemäße Vorrichtung dient der endovaskulären Denervation hirnversorgender Arterien zur Vorbeugung und Behandlung eines blutungsbedingten Vasospasmus. Die Vorrichtung ist in besonderer Weise flexibel und kann daher bis in Arterien im Schädelinneren eingeführt werden. Durch die Vorrichtung wird der arterielle Blutfluss nur so wenig verändert, dass eine Minderversorgung des Gehirns nicht zu befürchten ist. Die Vorrichtung kann einmalig zum Einsatz kommen, über mehrere Tage im zu behandelnden Gefäß verbleiben oder dauerhaft implantiert werden.

Die erfindungsgemäße Wirkung der Vorrichtung beruht auf einer Funktionsminderung oder -unterbrechung von Nervenfasern in der Gefäßwand der betroffenen Blutgefäße. Diese kann von einer vorübergehenden Funktionsminderung bis hin zu einer dauerhaften Zerstörung der Nervenfasern gehen. Zur Minderung/Unterbrechung der Funktion der Nervenfasern wird Energie von der Vorrichtung auf die Gefäßwand übertragen, wobei die Energieübertragung mittels elektrischer Impulse, Hochfrequenzimpulse oder Ultraschallimpulse erfolgen kann. Diese haben eine zumindest teilweise Verödung der Nervenfasern zur Folge. Die Energieübertragung auf die Gefäßwand erfolgt mit Hilfe von Elektroden bzw. Ultraschallsendern, wobei die Zuführung der Energie zu den Elektroden/Ultraschallsendern mittels elektrischer Leiter erfolgt, die Bestandteil der Stentstruktur sind. Die Elektroden, die in der Regel die Enden der elektrischen Leiter darstellen, sind üblicherweise gegenüber dem Leiter erweitert. Beispielsweise können die elektrischen Leiter runde oder eckige erweiterte Endabschnitte aufweisen, die als Elektroden fungieren.

Bei der Stentstruktur handelt es sich um eine selbstexpandierende Stentstruktur, d. h. sie nimmt nach Freisetzung aus einem Mikrokatheter, in dem sie an den Zielort vorgeschoben wird, selbstständig einen expandierten Zustand an, in dem sie sich an die Innenwandung des betroffenen Blutgefäßes anlegt. Darüber hinaus sollte der Übergang von komprimiertem zu expandiertem Zustand reversibel sein, d.h. die Stentstruktur sollte sich aus dem expandierten Zustand wieder in den komprimierten Zustand überführen lassen, insbesondere um sie nach Gebrauch in den Mikrokatheter zurückziehen und aus dem Blutgefäßsystem entfernen zu können. Derartige selbstexpandierende Stentstrukturen sind grundsätzlich aus dem Stand der Technik hinlänglich bekannt, beispielsweise zur dauerhaften Offenhaltung von Blutgefäßen im Falle von Gefäßverengungen bedingt durch Arteriosklerose. Ein Vorteil einer selbstexpandierenden Stentstruktur liegt darin, dass diese besonders filigran ausgestaltet sein kann, da zusätzliche Mittel zur Aufweitung wie Ballons verzichtbar sind. Typischerweise werden selbstexpandierende Stentstrukturen aus einem Material mit Formgedächtniseigenschaften hergestellt, insbesondere Formgedächtnismetallen, beispielsweise Nickel-Titan-Legierungen. Besonders häufig wird in diesem Zusammenhang Nitinol verwendet. Denkbar sind jedoch auch Polymere mit Formgedächtniseigenschaften oder andere Legierungen.

Die Einführhilfe ist typischerweise ein Einführdraht, auch Führungsdraht genannt. In ähnlicher Weise werden solche Einführdrähte auch für Implantate verwendet; allerdings ist bei Implantaten, die für den dauerhaften Verbleib im Gefäßsystem vorgesehen sind, der Einführdraht über eine Ablösestelle mit dem Implantat verbunden, wobei die Ablösestelle für eine mechanische, thermische, elektrolytische oder chemische Ablösung vorgesehen sein kann. Die Vorrichtung gemäß der Erfindung wird hingegen in der Regel nur temporär an die Zielposition gebracht, um dort eine Energieapplikation auf die Gefäßwand vorzunehmen. Die Einführhilfe ist bevorzugt aus Edelstahl, Nitinol oder einer Cobalt-Chrom-Legierung gefertigt. Denkbar ist jedoch auch eine Vorrichtung mit einer Stentstruktur, die dauerhaft im Gefäßsystem platziert werden kann, bei der also eine Ablösestelle zwischen Einführhilfe und Stentstruktur vorgesehen ist. In der Regel ist die Stentstruktur an ihrem proximalen Ende mit der Einführhilfe verbunden, auch wenn anderweitige Verbindungen zwischen Einführhilfe und Stentstruktur nicht ausgeschlossen sind.

Die Einführhilfe bzw. der Einführdraht ist bevorzugt am proximalen Ende der Stentstruktur radial außen angebracht. Mit anderen Worten befindet sich die Verbindung von Einführhilfe und Stentstruktur nicht im Zentrum der Stentstruktur, sondern exzentrisch an oder nahe der Gefäßinnenwandung. Auf diese Weise wird der Blutfluss möglichst wenig behindert. Die exzentrische Anordnung der Einführhilfe erleichtert darüber hinaus das Rückziehen der Vorrichtung in den Mikrokatheter.

In der Regel erfolgt die Behandlung in der Weise, dass die erfindungsgemäße Vorrichtung innerhalb eines Mikrokatheters an den Zielort, d. h. den Ort des Vasospasmus bzw. den Ort, an dem die Gefahr des Auftretens eines Vasospasmus besteht, vorgeschoben wird. Durch Zurückziehen des Mikrokatheters in Richtung proximal wird anschließend die Stentstruktur freigesetzt, die daraufhin expandiert und sich an die Innenwandung des Gefäßes anlegt. Anschließend erfolgt die Applikation von Impulsen auf die Nervenfasern in der Gefäßwand. Diese kann wiederholt erfolgen, auch über längere Zeiträume von mehreren Stunden oder mehreren Tagen. Schließlich wird der Mikrokatheter wieder in Richtung distal bewegt, um die Stentstruktur einzufalten, und der Mikrokatheter mitsamt der Vorrichtung zurückgezogen. Die beschriebene Behandlung kann an mehreren aufeinanderfolgenden Tagen wiederholt werden.

Die Begriffe "proximal" und "distal" sind so zu verstehen, dass beim Einbringen der Vorrichtung zum behandelnden Arzt weisende Teile als proximal, vom behandelnden Arzt weg weisende Teile als distal bezeichnet werden. Die Vorrichtung wird somit typischerweise durch einen Mikrokatheter in distaler Richtung vorgeschoben. Der Begriff "axial" bezieht sich auf die von proximal nach distal verlaufende Längsachse der Vorrichtung, der Begriff "radial" auf hierzu senkrechte Ebenen.

Parallel zu dieser Behandlung mit Hilfe der erfindungsgemäßen Vorrichtung kann auch eine medikamentöse Behandlung erfolgen, beispielsweise mit Nimodipin. Dieses kann insbesondere intraarteriell an der Stelle appliziert werden, an der ein Vasospasmus behandelt bzw. einem Vasospasmus vorgebeugt werden soll.

Grundsätzlich kann sich die Stentstruktur aus einzelnen, miteinander verbundenen Streben zusammensetzen. Eine solche Stentstruktur kann durch Laserschneiden in bekannter Weise hergestellt werden. Zusätzlich ist es sinnvoll, die Stentstruktur einer Elektropolitur zu unterziehen, um sie glatter und abgerundeter und damit weniger traumatisch werden zu lassen. Darüber hinaus sinkt die Gefahr der Anhaftung von Keimen oder sonstigen Verunreinigungen.

Alternativ kann die Stentstruktur auch eine Maschenstruktur aus einzelnen Drähten sein, die ein Geflecht ausbilden. Die Drähte verlaufen hierbei typischerweise helixförmig entlang der Längsachse, wobei gegenläufig verlaufende Drähte an den Kreuzungspunkten über- und untereinander her verlaufen, sodass sich wabenförmige Öffnungen zwischen den Drähten ausbilden. Die Gesamtzahl der Drähte beträgt bevorzugt 8 bis 64. Bei den Drähten, die die Maschenstruktur ausbilden, kann es sich um einzelne Drähte aus Metall handeln, möglich ist aber auch das Vorsehen von Litzen, d. h. mehreren Drähten geringen Durchmessers, die zusammen ein Filament bilden und vorzugsweise miteinander verdrillt sind.

Ein Vorteil einer Stentstruktur aus miteinander verbundenen Streben, die insbesondere durch Laserschneiden erzeugt wird, gegenüber einer Maschenstruktur aus Drähten ist darin zu sehen, dass eine Stentstruktur aus Streben bei Expansion weniger zur Längenkontraktion neigt als eine Maschenstruktur. Die Längenkontraktion sollte möglichst klein gehalten werden, da die Stentstruktur während einer Längenkontraktion die umgebende Gefäßwand zusätzlichem Stress aussetzt. Da ein Vasospasmus letztlich gerade auf Reize, die auf das Gefäß ausgeübt werden, zurückzuführen ist, ist eine zusätzliche Belastung bei der Vasospasmusbehandlung zu vermeiden.

Die Streben oder Drähte können einen runden, ovalen, quadratischen oder rechteckigen Querschnitt aufweisen, wobei im Falle eines quadratischen oder rechteckigen Querschnitts eine Abrundung der Kanten von Vorteil ist. Bei Verwendung von Streben oder Drähten mit einem im Wesentlichen rechteckigen Querschnitt haben sich eine Höhe und eine Breite der Streben/Drähte von 20 bis 300 µm, bevorzugt 20 bis 70 µm als vorteilhaft herausgestellt, wobei auch ein rechteckiger Querschnitt mit abgerundeten Kanten als im Wesentlichen rechteckig aufgefasst wird. Im Falle eines runden Querschnitts sollte der Durchmesser zwischen 20 und 300 µm, bevorzugt zwischen 20 und 70 µm liegen. Unabhängig davon, ob Streben oder geflochtene Drähte vorliegen, ist von Bedeutung, dass elektrische Leiter vorgesehen sind, um die Applikation von Impulsen auf die Nervenfasern durchführen zu können. Bei den elektrischen Leitern kann es sich um die Streben/Drähte selbst handeln, die Leiter können mit den Streben/Drähten verbunden sein oder die Leiter sind separate Bestandteile der Stentstruktur.

Gemäß der Erfindung weist die Stentstruktur ein von proximal nach distal verlaufendes Rückgrat auf, von dem Streben ausgehen, die im expandierten Zustand den Umfang der Stentstruktur ausbilden. Die Stentstruktur kann beispielsweise einem menschlichen Rückgrat ähneln, wobei die vom Rückgrat ausgehenden Streben den Rippen entsprechen. Insbesondere können die vom Rückgrat ausgehenden Streben im Wesentlichen einen Ring ausbilden, wenn sie im expandierten Zustand vorliegen, so dass sie über den gesamten oder große Teile des Umfangs an der im Querschnitt betrachtet im Wesentlichen kreisförmigen Innenwandung des Blutgefäßes anliegen.

Insbesondere können jeweils zwei Streben offene Ringe ausbilden, die einen Spalt aufweisen. Die Verbindungspunkte zwischen den Streben und dem Rückgrat können auch gegeneinander versetzt sein; dies vermindert das Risiko, dass sich elektrische Leiter der Streben berühren und einen Kurzschluss erzeugen.

Die Streben, die vom Rückgrat ausgehen und offene Ringe ausbilden, können auch aus zwei oder mehr Teilstreben aufgebaut sein, d. h. zwei oder mehr vom Rückgrat ausgehende Teilstreben verlaufen parallel zu einander und enden in einem gemeinsamen Endpunkt. Zwischen den Endpunkten, die durch einander gegenüberliegende Gruppen von Teilstreben gebildet werden, ergibt sich somit ein Spalt. Sofern sich eine Strebe aus zwei Teilstreben zusammensetzt, kann man die Ausführungsform auch in der Weise beschreiben, dass die beiden Teilstreben gemeinsam einen Bogen am Rückgrat ausbilden, wobei der Scheitelpunkt des Bogens dem zuvor erwähnten Endpunkt entspricht.

Unabhängig von der Ausführungsform, bei der die Streben von einem gemeinsamen Rückgrat ausgehen, können auch im Übrigen Streben aus parallel verlaufenden Teilstreben aufgebaut sein. Eine Stentstruktur mit mehreren schmaleren Teilstreben kann sich zuverlässiger radial entfalten als eine Stentstruktur mit breiteren Streben, wenn die Stentstruktur vom äußeren Zwang des Mikrokatheters befreit wird.

Der Winkel, den die vom Rückgrat ausgehenden Streben gegenüber dem Rückgrat ausbilden, kann ein rechter Winkel sein, es sind jedoch auch Abweichungen vom rechten Winkel möglich, beispielsweise eine Erstreckung in gewissem Maße in Richtung proximal oder distal. Der an der Verbindungsstelle von Streben und Rückgrat vorliegende Winkel kann somit im expandierten Zustand insbesondere zwischen 30° und 90° liegen, wobei die Streben sowohl nach distal als auch nach proximal weisen können. Typischer sind allerdings Ausführungsformen, bei denen die Streben in Richtung distal weisen. Die Streben können elektrische Leiter aufweisen, um die Applikation eines Impulses auf die Nervenfasern durchführen zu können. Es kann dabei ausreichend sein, wenn lediglich einzelne vom Rückgrat ausgehende Streben elektrische Leiter aufweisen; ebenso gut ist aber die Ausstattung sämtlicher Streben mit elektrischen Leitern möglich. Die Anzahl der vom Rückgrat ausgehenden Streben kann ebenfalls unterschiedlich gewählt werden, die Mindestzahl der Streben beträgt somit eins.

Die elektrischen Leiter sollten am proximalen Ende der Stentstruktur zusammenlaufen und mit der Einführhilfe verbunden sein. Über die Länge der Einführhilfe besteht in der Regel eine elektrische Verbindung der elektrischen Leiter bis zu einer Stromquelle, die typischerweise außerhalb des Körpers liegt. Auf diese Weise wird sichergestellt, dass elektrische oder andere Impulse von extern gesteuert durch die Stentstruktur ausgeübt werden können. Grundsätzlich denkbar wäre allerdings auch eine Stromquelle, die Bestandteil der Vorrichtung selbst ist, in diesem Fall müsste die Stromquelle jedoch besonders kompakt sein, um in intrakranielle Blutgefäße eingeführt werden zu können. Die Zahl der elektrischen Leiter kann variieren, je nachdem, ob lediglich ein einzelnes Elektrodenpaar/ein Ultraschallsender oder mehrere Elektrodenpaare/Ultraschallsender vorgesehen sind. Auf der einen Seite ist das Vorsehen mehrerer elektrischer Leiter insofern von Vorteil, als auf diese Weise an verschiedenen Stellen der Gefäßinnenwandung Impulse appliziert werden können, ggf. auch gleichzeitig, auf der anderen Seite muss darauf geachtet werden, dass die gesamte Vorrichtung noch hinreichend flexibel bleibt, um in englumige, intrakranielle Blutgefäße vorgeschoben werden zu können.

Die einzelnen elektrischen Leiter sollten einander gegenüber elektrisch isoliert sein, um keine Kurzschlüsse zu herbeizuführen. Dies gilt insbesondere dann, wenn die die Stentstruktur ausbildenden Streben oder Drähte relativ eng zueinander verlaufen. Unter Umständen kann es ausreichend sein, wenn elektrische Leiter lediglich in den Bereichen, in denen sie eng aneinander vorbei geleitet werden, elektrisch isoliert sind, beispielsweise am proximalen Ende der Stentstruktur, an dem die elektrischen Leiter zur Einführhilfe übergehen, grundsätzlich ist es jedoch von Vorteil, wenn eine vollständige Isolierung der elektrischen Leiter vorliegt, ggf. abgesehen von den Bereichen der elektrischen Leiter, über die die Impulsapplikation erfolgt, d. h. in der Regel den Enden, an denen die Elektroden angeordnet sind.

Möglich ist auch eine Ausführungsform, bei der die elektrischen Leiter elektrisch isoliert sind, jedoch an einigen Stellen bewusst keine elektrische Isolierung vorgesehen ist, sodass von diesen Stellen bzw. zwischen diesen Stellen Impulse ausgehen können. Eine solche Ausführungsform ist insbesondere geeignet für eine Stentstruktur, die eine Maschenstruktur aus einzelnen Drähten aufweist, welche ein Geflecht ausbilden. Die Expansion der Stentstruktur sorgt in diesem Fall quasi automatisch dafür, dass die Drähte, von denen zumindest einige gleichzeitig eine Funktion als elektrische Leiter erfüllen können, an der Innenwandung des Gefäßes anliegen, somit auch die Stellen der elektrischen Leiter, an denen auf eine Isolierung verzichtet wurde. Ein Vorteil einer solchen Stentstruktur ist, dass sie für verschiedene Blutgefäße mit unterschiedlichen Durchmessern Verwendung finden kann.

Ein weiteres Merkmal der Erfindung ist eine Vorrichtung, bei der Paare von elektrischen oder Hochfrequenz(HF)-Elektroden auf dem Umfang der Stentstruktur in einer Weise angeordnet sind, dass die Elektroden im expandierten und in das Blutgefäß implantierten Zustand durch einen Spalt voneinander beabstandet sind, so dass ein applizierter Stromfluss zu den Elektroden über den Spalt hinweg auf die Innenwandung des Blutgefäßes einwirkt. Bei dem Impuls kann es sich um einen elektrischen oder einen Hochfrequenzimpuls handeln. Die Ausführungsform wird erfindungsgemäß mit der zuvor beschriebenen Ausführungsform kombiniert, bei der die Stentstruktur ein von proximal nach distal verlaufendes Rückgrat aufweist, von dem elektrische Leiter aufweisende Streben ausgehen. So können vom Rückgrat Strebenpaare ausgehen, an deren Enden jeweils die Elektroden angeordnet sind. Angesichts der geringen Größe der Stentstruktur insgesamt ist naturgemäß der Abstand der Elektroden ebenfalls klein und liegt in der Regel bei ≤ 1 mm.

Sinnvoll ist darüber hinaus, wenn die Elektroden über eine röntgendichte Markierung verfügen. Auf diese Weise kann der behandelnde Arzt erkennen, ob die Elektroden noch, wie gewünscht, einen geringen Abstand voneinander haben oder aber sich berühren, d. h. ein Kurzschluss entsteht. Da die Streben auch im expandierten Zustand einer von der Gefäßinnenwandung ausgehenden, radial wirkenden Kraft unterliegen, hängt es auch vom Innendurchmesser des Blutgefäßes ab, wie sehr die Stentstruktur komprimiert wird. Entsprechend kann beispielsweise eine bestimmte Stentstruktur bei einem ausreichend großen Blutgefäß verwendbar sein, weil die Elektroden einen hinreichend großen Spalt zwischen sich aufweisen, bei kleineren Blutgefäßen kann hingegen ein Kontakt zwischen den Elektroden aufgrund der stärkeren Komprimierung der Stentstruktur vorliegen, weshalb keine Impulsapplikation möglich ist. Für Blutgefäße mit unterschiedlichem Innendurchmesser sollten daher unterschiedliche Stentstrukturen vorgehalten werden.

Möglich ist auch eine Ausführungsform bei der zwischen den Elektroden eine Brücke aus isolierendem Material vorgesehen ist. Auf diese Weise wird ein Kurzschluss wirkungsvoll verhindert. Wenn das isolierende Material zudem eine gewisse Flexibilität aufweist, kann sich die Stentstruktur an den Innendurchmesser des Blutgefäßes anpassen. Auf diese Weise kann eine bestimmte Stentstruktur in unterschiedlich großen Blutgefäßen eingesetzt werden.

Besonders vorteilhaft ist es, wenn die Elektroden innen einen röntgendichten Kern aufweisen, der als Markierung dienen kann. Auch an anderen Positionen der Vorrichtung können ein oder mehrere röntgendichte Markierungen vorhanden sein, um dem behandelnden Arzt eine Visualisierung der Platzierung und Entfaltung der Vorrichtung zu ermöglichen. Die röntgendichten Markierungen können z. B. aus Platin, Palladium, Platin-Iridium, Tantal, Gold, Wolfram oder anderen röntgendichten Metallen sein. Besonders sinnvoll sind entsprechende röntgendichte Markierungen an den Enden der Stentstruktur, insbesondere am distalen Ende. Möglich ist auch, die Streben oder Drähte der Stentstruktur mit einer Beschichtung aus einem röntgendichten Material zu versehen, beispielsweise mit einer Goldbeschichtung. Diese kann z. B. eine Stärke von 1 bis 6 µm aufweisen. Eine derartige Goldbeschichtung kann zusätzlich oder anstelle der röntgendichten Markierungen verwendet werden.

Sinnvollerweise verfügt die Stentstruktur über mehrere Paare von Elektroden, die einen elektrischen oder Hochfrequenz-Impuls erzeugen können. Auf diese Weise kann an mehreren Stellen der Gefäßwandung ein Impuls appliziert werden, wobei die Applikation zeitgleich oder nacheinander erfolgen kann. Dies ist insofern von Bedeutung, als die Gefäßwandung häufig mehrere Nervenfasern aufweist, deren Denervation für den Behandlungserfolg von Bedeutung ist.

Die Elektroden, Paare von Elektroden und/oder Ultraschallsender (allgemein: Impulsgeber) können in Längsrichtung der Stentstruktur betrachtet auf dem Umfang gegeneinander versetzt angeordnet sein. Mit anderen Worten wirken die Elektroden, die von proximal nach distal in verschiedenen Segmenten der Stentstruktur angeordnet sind, auch hinsichtlich des Innenumfangs des Blutgefäßes auf unterschiedliche Abschnitte. Im Querschnitt betrachtet kann beispielsweise ein Impulsgeber auf 12:00 Uhr, ein Impulsgeber auf 3:00 Uhr, ein Impulsgeber auf 6:00 Uhr und ein weiterer Impulsgeber schließlich auf 9:00 Uhr-Position stehen. Eine solche Ausführungsform hat den Vorteil, dass, ohne die Stentstruktur rotieren zu müssen, unterschiedliche in der Gefäßwandung in Längsrichtung verlaufende Nervenfasern erfasst werden können. Ggf. kann die Stentstruktur nach distal vorgeschoben bzw. proximal zurückgezogen werden, um gezielt bestimmte Elektrodenpaare auf verschiedene Umfangspositionen der Gefäßwandung zu bringen und dort die Impulse einwirken zu lassen. Dies ist insofern von Bedeutung, als ein Vorschieben bzw. Zurückziehen der Vorrichtung und damit der Stentstruktur vergleichsweise einfach, eine Rotation der Stentstruktur jedoch nur schwierig zu erreichen ist, da die Vorrichtung in der Regel über erhebliche Entfernungen bis in den intrakraniellen Bereich vorgeschoben wird, was die Übertragung von Torsionskräften erheblich erschwert.

Von Vorteil ist eine Ausführungsform, bei der die Stentstruktur mehrere, in Längsrichtung der Vorrichtung beabstandete, im Wesentlichen ringförmige Elemente aufweist, die jeweils zwei zu einem Stromkreis gehörende elektrische Leiter aufweisen, wobei die beiden elektrischen Leiter jeweils in einer Elektrode enden und die beiden Elektroden durch einen Spalt voneinander getrennt sind, wenn sich die Vorrichtung im expandierten Zustand implantiert in das Blutgefäß befindet. Die genannte Ausführungsform lässt sich insbesondere mit der zuvor beschriebenen Ausführungsform kombinieren, bei der Streben von einem von proximal nach distal verlaufenden Rückgrat ausgehen, wobei im vorliegenden Fall eine Strebe vom Rückgrat in einer ersten Richtung mit einem ersten elektrischen Leiter und eine zweite Strebe in einer zweiten Richtung mit einem zweiten elektrischen Leiter ausgeht. Die Stentstruktur ähnelt somit einem menschlichen Rückgrat mit Rippen, wobei die Enden durch einen Spalt voneinander getrennt sind. Es handelt sich somit nicht um eine geschlossene, sondern eine offene Ringform. Wie zuvor beschrieben, können die Spalte zwischen den einzelnen Streben für unterschiedliche Strebenpaare versetzt zueinander angeordnet sein. Möglich ist auch die Ausfüllung der Spalte mit einem elektrisch isolierenden Material.

Zweckmäßigerweise sollte die Vorrichtung, bevorzugt die Stentstruktur, Mittel zur Messung von elektrischen Widerständen aufweisen, insbesondere Mittel zur Impedanzmessung, d. h. der Messung von Wechselstromwiderständen. Eine solche Widerstandsmessung ist insofern von Bedeutung, als unterschiedliche Gewebe einen unterschiedlichen elektrischen Widerstand aufweisen können. Um bestimmen zu können, welche Energie zur Denervation bestimmter Nervenfasern aufgewendet werden muss, ist daher eine Widerstandsmessung sinnvoll. Auf Basis des bestimmten Widerstands kann beispielsweise mit Hilfe einer Datenmatrix bestimmt werden, mit welchem definierten Strom-Spannungssignal der gewünschte Effekt, beispielsweise die Herbeiführung einer bestimmten Temperatur, erzielbar ist. Nach der Behandlung kann mit einer weiteren Widerstandsmessung der Erfolg der Behandlung kontrolliert werden.

Die Widerstandsmessung muss nicht zwangsläufig in die erfindungsgemäße Vorrichtung integriert sein, denkbar ist auch eine separate Vorrichtung zur Widerstandsmessung.

Die Stentstruktur kann durchlässig sein, d. h. in radialer Richtung Öffnungen aufweisen, es ist jedoch auch möglich, eine Stentstruktur vorzusehen, die auf der Innenseite, d.h. der luminalen Seite eine Membran aufweist. Auf der abluminalen Seite tritt die Oberfläche der Vorrichtung hingegen in direkten Kontakt mit der Gefäßinnenwand. In diesem Fall ist das Gefäßlumen von den in der Regel metallischen Drähten oder Streben der Stentstruktur mittels der Membran abgekoppelt. Die Membran kann darüber hinaus eine gewisse elektrische Isolierung der elektrischen Leiter in luminaler Richtung erzeugen. Auf der anderen Seite erfordert das Vorsehen einer zusätzlichen Membran bei Vorliegen der Stentstruktur im komprimierten Zustand zusätzlichen Raum, sodass eine solche Stentstruktur weniger eng zusammenfaltbar ist.

In der Regel ist die Stentstruktur am proximalen Ende offen ausgebildet. Am distalen Ende kann die Stentstruktur ebenfalls offen, aber auch geschlossen sein. Eine beidseitig offene Stentstruktur hat den Vorteil, dass der Blutfluss möglichst wenig gestört und einer Unterversorgung nachfolgender Blutgefäße und des durch diese versorgten Gewebes vorgebeugt wird. Auf der anderen Seite ist eine geschlossene Struktur am distalen Ende atraumatischer. Unter offen wird dabei verstanden, dass am jeweiligen Ende der Stentstruktur keine Streben oder Drähte vorhanden sind und sich Streben/Drähte auf den äußeren Umfang der Stentstruktur beschränken. Bei einem geschlossenen Ende hingegen liegen auch im Zentrum der Stentstruktur Streben oder Drähte vor. Da zwischen den Streben oder Drähten Öffnungen vorhanden sind, ist auch bei einem geschlossenen distalen Ende dieses Ende nicht vollständig dicht; der Blutfluss kann weiterhin durch die Öffnungen hindurch erfolgen.

Sinnvoll ist eine antithrombogene Beschichtung der Stentstruktur. Diese kann die gesamte Stentstruktur oder auch lediglich die Innenseite betreffen, weil die Stentstruktur über eine gewisse Zeit im Blutgefäß verbleibt und währenddessen die Bildung eines Thrombus im ohnehin durch das Auftreten des Vasospasmus verengten Blutgefäß vermieden werden muss. Auf der Außenseite der Stentstruktur ist eine gefäßrelaxierende Beschichtung von Vorteil, beispielsweise mit einem Calciumkanalblocker wie Nimodipin.

Der Durchmesser der Stentstruktur im frei expandierten Zustand liegt typischerweise im Bereich von 2 bis 8 mm, vorzugsweise im Bereich von 4 bis 6 mm. Die Gesamtlänge der Stentstruktur im expandierten Zustand beträgt in der Regel 5 bis 50 mm, bevorzugt 10 bis 45 mm, weiter bevorzugt 20 bis 40 mm. Im Falle einer Stentstruktur aus Streben kann diese beispielsweise aus einem Rohr mit einer Wandstärke von 25 bis 70 µm geschnitten werden; im Falle einer Maschenstruktur aus miteinander verflochtenen Drähten beträgt die Drahtdicke bevorzugt 20 bis 70 µm. Ein Mikrokatheter, durch den die Vorrichtung im komprimierten Zustand an den Zielort gebracht werden kann, hat z. B. einen Innendurchmesser von 0,4 bis 0,9 mm.

Die erfindungsgemäße Vorrichtung findet Anwendung in einem Verfahren zur Vorbeugung oder Behandlung eines Vasospasmus. Dabei wird die Stentstruktur der erfindungsgemäßen Vorrichtung mit der Einführhilfe an die Zielposition im Blutgefäß gebracht und dort expandiert, was in der Regel durch Zurückziehen des Mikrokatheters, in dem die Vorrichtung untergebracht ist, in Richtung proximal erfolgt. Anschließend werden elektrische Impulse, Hochfrequenzimpulse oder Ultraschallimpulse auf in der Gefäßwand des Blutgefäßes verlaufende Nervenfasern appliziert. Die Impulsapplikation kann bei Bedarf mehrfach wiederholt werden. Hierbei kann die Stentstruktur zwischen den einzelnen Impulsapplikationen an einer gegebenen Position bleiben, nach distal vorgeschoben oder nach proximal zurückgezogen werden, um bei Bedarf unterschiedliche Nervenfasern zu erfassen. Das Verschieben der Stentstruktur erfolgt in aller Regel innerhalb des Mikrokatheters, da insbesondere beim Vorschub der expandierten Stentstruktur die Gefahr der Verletzung von Blutgefäßen zu hoch wäre. Eine Verletzung oder zu starke Reizung sollte in jedem Fall vermieden werden, da diese kausal für das Auftreten eines Vasospasmus sein kann. Ein Vorschub oder Zurückziehen der Stentstruktur in eine andere Längsposition erfolgt somit in der Weise, dass zunächst der Mikrokatheter vorgeschoben wird, um die Stentstruktur in den komprimierten Zustand zu überführen, wobei die Stentstruktur vom Mikrokatheter aufgenommen wird, der Mikrokatheter und damit auch die innerhalb des Mikrokatheters befindliche Stentstruktur an die gewünschte Position gebracht wird und die Stentstruktur schließlich erneut aus dem Mikrokatheter freigesetzt wird.

Die gesamte Vorrichtung kann auch zwischenzeitlich aus dem Blutgefäßsystem entfernt und später erneut eingeführt werden, um die Behandlung beispielsweise über mehrere Tage fortführen zu können. Aus Sterilitätsgründen muss dabei jedoch normalerweise für jede Behandlung eine neue Vorrichtung verwendet werden. Die Entfernung der Vorrichtung aus dem Blutgefäßsystem erfolgt in der Regel in der Weise, dass der Mikrokatheter in Richtung distal über die freigesetzte Stentstruktur geschoben wird, woraufhin diese sich erneut zusammenfaltet und zusammen mit dem Mikrokatheter nach proximal zurückgezogen werden kann.

Sämtliche Ausführungen, die bezüglich der Vorrichtung gemacht wurden, gelten in gleicher Weise auch für das Verfahren und umgekehrt.

Die Erfindung wird durch die beiliegenden Abbildungen beispielhaft näher erläutert. Es zeigen:
- Figur 1: Eine erfindungsgemäße Vorrichtung in der Seitenansicht;
- Figur 2: die Stentstruktur der erfindungsgemäßen Vorrichtung aus Figur 1;
- Figur 3: einen Ausschnitt aus Figur 2;
- Figur 4: eine alternative Stentstruktur;
- Figur 5: einen Ausschnitt aus einer Stentstuktur mit mehreren elektrischen Leitern;
- Figur 6: Elektroden der Stentstruktur und
- Figur 7: eine Stentstruktur in abgewickelter Form.

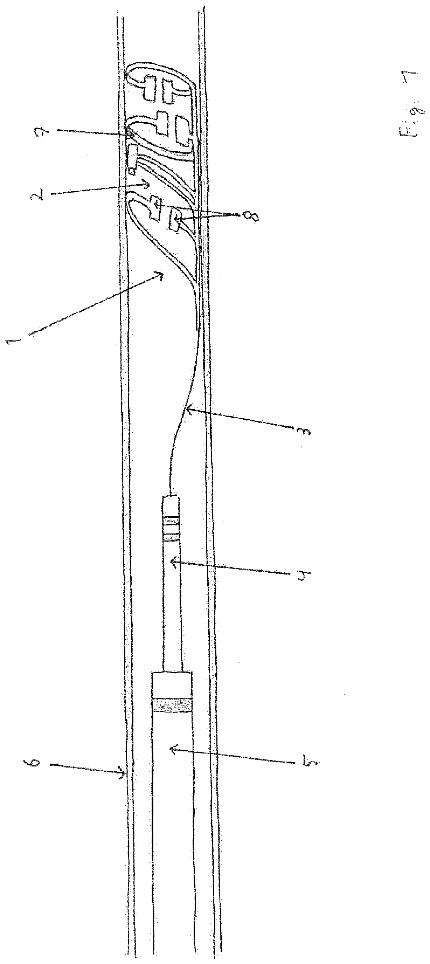

In Figur 1 ist eine erfindungsgemäße Vorrichtung 1 innerhalb eines Blutgefäßes 6 in der Seitenansicht dargestellt. Die Vorrichtung 1 weist eine Stentstruktur 2 und eine Einführhilfe 3 auf, bei der es sich um einen Führungsdraht handelt. Die Stentstruktur 2 ist hier in ihrer expandierten, in das Blutgefäß 6 implantierten Form dargestellt. Der Vorschub der Stentstruktur 2 erfolgt innerhalb des Mikrokatheters 4 von proximal (hier: links) nach distal (hier: rechts); durch Vorschieben des Mikrokatheters 4 oder Zurückziehen der Stentstruktur 2 kann diese sich wieder so eng zusammenfalten, dass sie in den Mikrokatheter 4 gelangt und zusammen mit diesem aus dem Blutgefäßsystem entfernt werden kann. Der Mikrokatheter 4 seinerseits wird durch einen weiteren Katheter 5 mit größerem Lumen geführt.

Die Stentstruktur 2 selbst weist Streben 7 auf, die jeweils paarweise im Wesentlichen ringförmig sind und sich an die Innenwandung des Blutgefäßes 6 anlegen. Darüber hinaus weisen die Streben 7 elektrische Leiter auf, die mit den Elektroden 8 am Ende der Streben 7 in elektrisch leitender Verbindung stehen. Es liegen für jeden Ring aus Streben 7 Paare zueinander gehöriger Elektroden 8 vor, zwischen denen ein kleiner Spalt vorgesehen ist, über den ein Impuls, beispielsweise ein elektrischer oder HF-Impuls auf das umliegende Gewebe appliziert werden kann. Des Weiteren erkennt man, dass für die einzelnen durch die Streben 7 ausgebildeten Ringe die Elektrodenpaare 8 und somit auch der Spalt zwischen den Elektroden hinsichtlich der Position im Umfang des Blutgefäßes gegeneinander versetzt sind, d. h. unterschiedliche Ringe von Streben 7 applizieren Impulse an unterschiedlichen radialen Positionen.

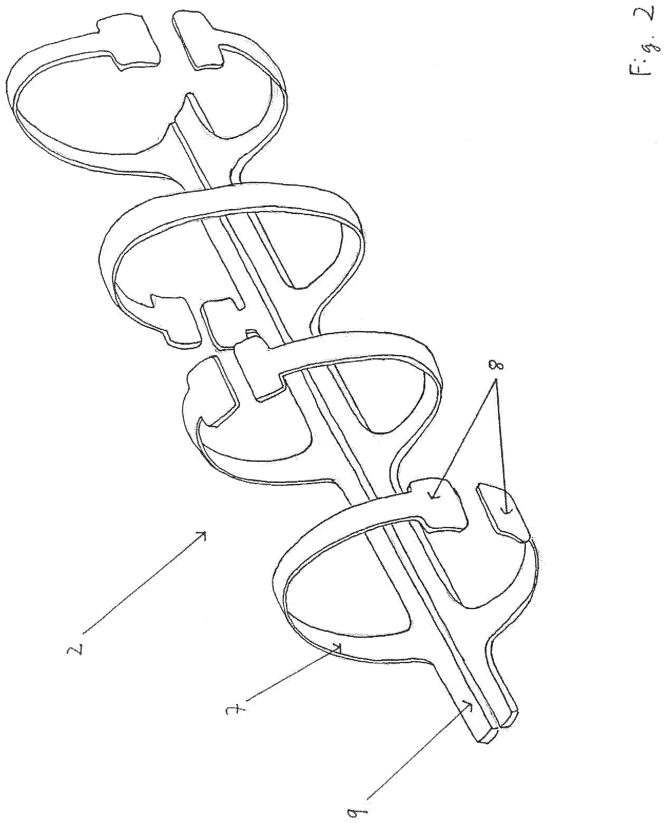

In Figur 2 ist die Stentstruktur 2 aus Figur 1 in einer vergrößerten Ansicht dargestellt. Man erkennt, dass die Streben 7 jeweils paarweise einen offenen Ring ausbilden, wobei sich jeweils am Ende einer Strebe 7 eine Elektrode 8 befindet. Für die einzelnen, in Längsrichtung hintereinander angeordneten Ringe von Streben 7 sind die Elektroden 8 jeweils gegeneinander versetzt angeordnet. Die Impulse, die auf unterschiedliche radiale Bereiche der Wandung des Blutgefäßes und die darin verlaufenen Nervenfasern einwirken sollen, können gleichzeitig, aber auch zeitlich versetzt abgegeben werden. Für einen bestimmten Ring von Streben 7 kann beispielsweise auch durch Längsverschiebung der Stentstruktur 2 ausgewählt werden, an welcher Stelle die Impulsapplikation erfolgen soll.

Die Streben 7 gehen von einem gemeinsamen Rückgrat 9 aus, das in Längsrichtung der Stentstruktur 2 verläuft. Dabei kann, wie hier dargestellt, das Rückgrat 9 selbst zweigeteilt sein, so dass die eine Hälfte des Rückgrats 9 der Stromzuführung zur ersten Hälfte der Streben 7, die zweite Hälfte des Rückgrats 9 der Stromzuführung zur zweiten Hälfte der Streben 7 dient.

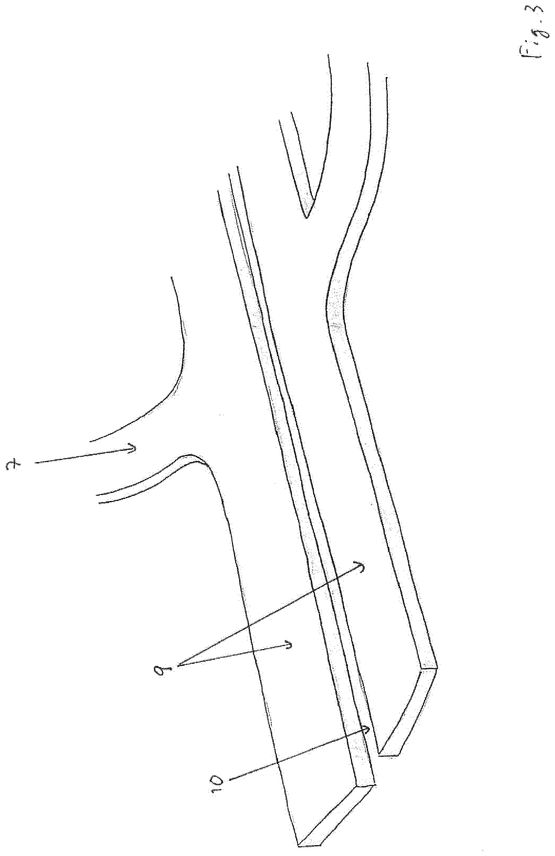

Ein Ausschnitt der Stentstruktur 2 aus Figur 2 ist in der Figur 3 dargestellt; man erkennt, wie die Streben 7 mit dem Rückgrat 9 verbunden sind und dass zwischen den beiden Hälften des Rückgrats 9 eine Isolierung 10 gegeben ist, die dafür sorgt, dass zwischen den beiden Hälften des Rückgrats 9 kein Kurzschluss auftritt.

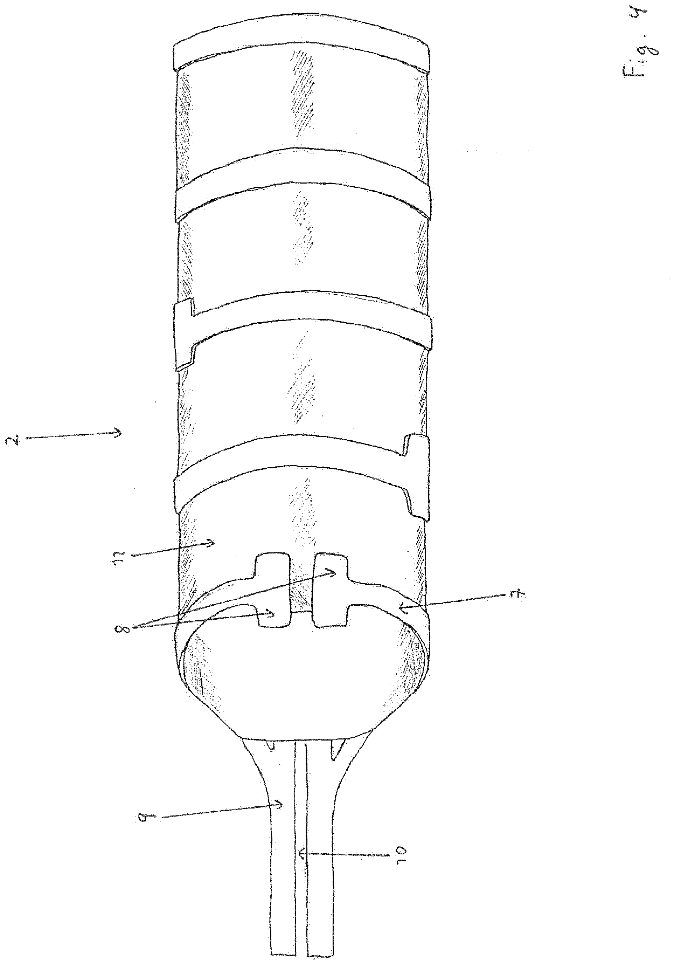

In Figur 4 ist eine Stentstruktur 2 dargestellt, die grundsätzlich der Stentstruktur 2 aus Figur 2 ähnlich ist, bei der jedoch auf der luminalen, d. h. Innenseite der Stentstruktur 2 eine Membran 11 vorgesehen ist, die das eigentliche Lumen des Blutgefäßes 6 von der Stentstruktur 2 trennt und somit eine Isolierung in luminaler Richtung herbeiführt.

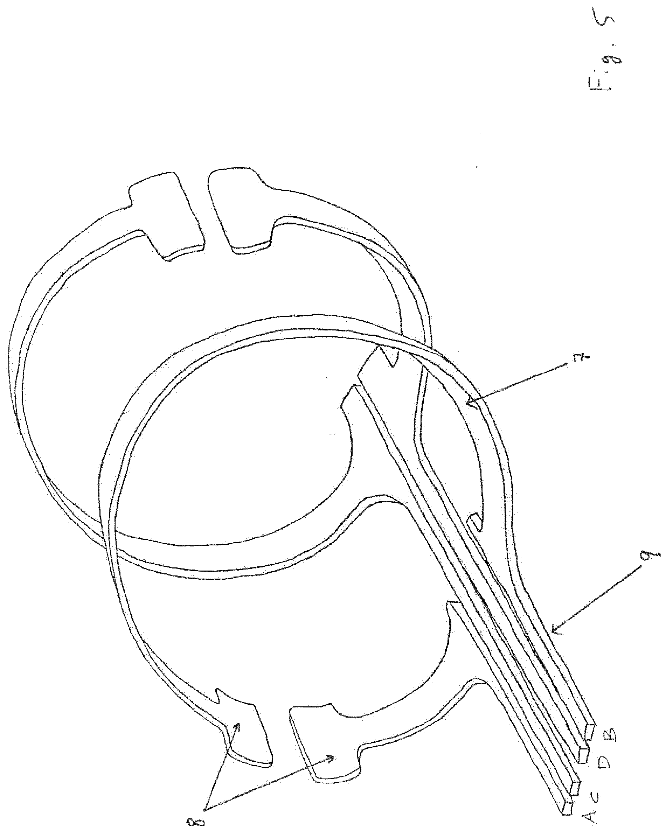

In Figur 5 sind zwei in Längsrichtung hintereinander angeordnete, von Streben 7 ausgebildete offene Ringe dargestellt, die jeweils vom Rückgrat 9 ausgehen. Letzteres verfügt über 4 Leiter A, B, C, D, die für eine Stromzuführung zu den Elektroden 8 sorgen. Die Stromzufuhr über die Leiter A, B einerseits und C, D andererseits kann gleichzeitig oder sequentiell erfolgen.

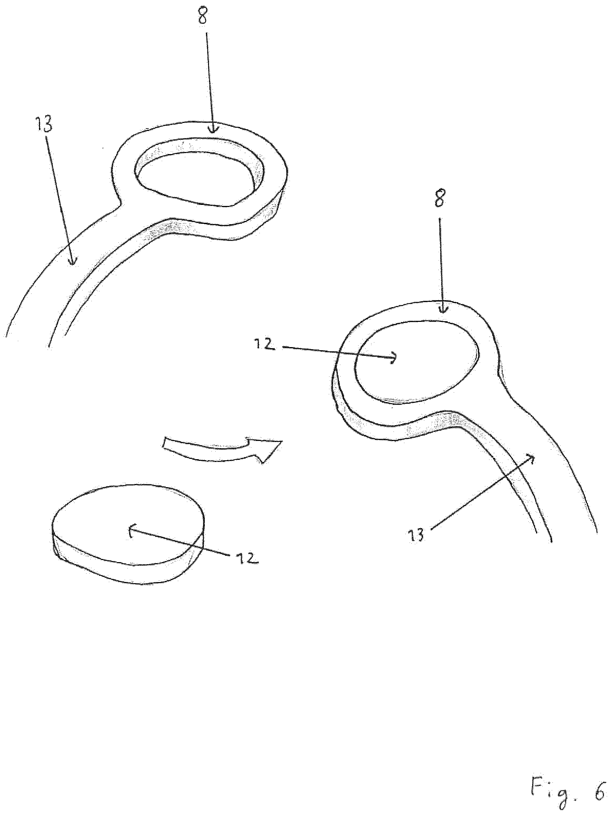

In Figur 6 sind Elektroden 8 gezeigt, die die Enden der elektrischen Leiter 13 bilden. In der hier gezeigten Ausführung wird der elektrische Strom unmittelbar über die Streben 7 zu den Elektroden 8 geführt, d. h. die Streben 7 sind zugleich elektrische Leiter 13. Zwecks Visualisierung der Elektroden 8 weisen diese innen eine Öffnung auf, in die Röntgenmarker 12 eingepresst werden. Die Röntgenmarker 12 können beispielsweise aus Platin oder einer Platinlegierung bestehen. Im Röntgenbild erkennt der behandelnde Arzt auf diese Weise unmittelbar, wie die die für die Behandlung wesentlichen Impulse herbeiführenden Elektroden 8 angeordnet sind. Darüber hinaus kann der Arzt prüfen, dass zwischen den Elektroden 8 kein Kurzschluss vorliegt.

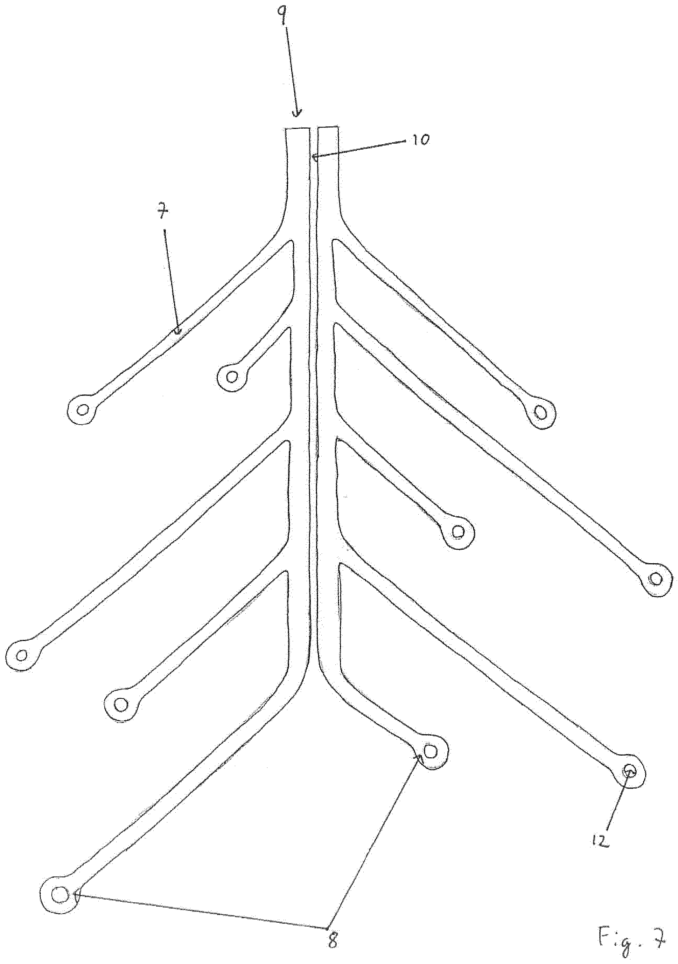

In Figur 7 ist eine Stentstruktur 2 in abgewickelter Form gezeigt, d. h. die einen offenen Ring ausbildenden Streben 7 wurden in eine Fläche gedrückt und sind hier zweidimensional dargestellt. Man erkennt, dass die Streben 7 unterschiedliche Längen aufweisen. Auf diese Weise wird erreicht, dass die Elektroden 8 letztlich nach Einbringung in das Blutgefäß auf der Innenwandung des Blutgefäßes versetzt angeordnet sind und sich Impulse auf unterschiedliche Abschnitte auswirken.

## Patentansprüche

1. Vorrichtung mit einer Stentstruktur (2), die zur Einbringung in intrakranielle Blutgefäße (6) des menschlichen oder tierischen Körpers vorgesehen ist, wobei die Stentstruktur (2) einen expandierten Zustand, in dem sie in der Lage ist, sich an die Innenwandung des Blutgefäßes (6) anzulegen, und einen komprimierten Zustand aufweist, in dem sie innerhalb eines Mikrokatheters (4) durch das Blutgefäß (6) bewegbar ist, wobei die Stentstruktur (2) mit einer Einführhilfe (3) verbunden ist und wobei die Stentstruktur (2) in der Lage ist, nach Freisetzung aus dem Mikrokatheter (4) selbständig in den expandierten Zustand überzugehen,
wobei die Stentstruktur (2) elektrische Leiter (13) aufweist, über die elektrische Impulse oder Hochfrequenzimpulse auf in der Gefäßwand des Blutgefäßes (6) verlaufende Nervenfasern applizierbar sind, um die Funktion der Nervenfasern vorübergehend oder dauerhaft zu mindern und einem Vasospasmus vorzubeugen oder diesen zu behandeln, und wobei die Stentstruktur (2) ein von proximal nach distal verlaufendes Rückgrat (9) aufweist, von dem elektrische Leiter (13) aufweisende Streben (7) ausgehen, die im expandierten Zustand den Umfang der Stentstruktur (2) ausbilden, wobei Paare von elektrischen oder Hochfrequenz-Elektroden (8), die an die elektrischen Leiter (13) angeschlossen sind, auf dem Umfang der Stentstruktur (2) in einer Weise angeordnet sind, dass die Elektroden (8) im expandierten und in das Blutgefäß (6) implantierten Zustand durch einen Spalt voneinander beabstandet sind, sodass ein applizierter Stromfluss zu den Elektroden (8) über den Spalt hinweg auf die Innenwandung des Blutgefäßes (6) einwirkt.

2. Vorrichtung nach Anspruch 1, **dadurch gekennzeichnet, dass** zumindest einige Streben (7) sich aus parallel zueinander verlaufenden Teilstreben zusammensetzen.

3. Vorrichtung nach Anspruch 1 oder 2, **dadurch gekennzeichnet, dass** die elektrischen Leiter (13) am proximalen Ende der Stentstruktur (2) zusammenlaufen und mit der Einführhilfe (3) verbunden sind.

4. Vorrichtung nach einem der Ansprüche 1 bis 3, **dadurch gekennzeichnet, dass** die elektrischen Leiter (13) gegeneinander elektrisch isoliert sind.

5. Vorrichtung nach einem der Ansprüche 1 bis 4, **dadurch gekennzeichnet, dass** die Elektroden (8) über eine röntgendichte Markierung (12) verfügen.

6. Vorrichtung nach einem der Ansprüche 1 bis 5, **dadurch gekennzeichnet, dass** die Stentstruktur (2) über mehrere Paare von Elektroden (8) verfügt.

7. Vorrichtung nach einem der Ansprüche 1 bis 6, **dadurch gekennzeichnet, dass** der Spalt zwischen einem Paar von Elektroden (8) mit einem elektrisch isolierenden Material ausgefüllt ist.

8. Vorrichtung nach einem der Ansprüche 1 bis 7, **dadurch gekennzeichnet, dass** die Stentstruktur (2) mehrere, in Längsrichtung der Vorrichtung (1) beabstandete, im Wesentlichen ringförmige Elemente aufweist, die jeweils zwei zu einem Stromkreis gehörende elektrische Leiter (13) aufweisen, wobei die beiden elektrischen Leiter (13) jeweils in einer Elektrode (8) enden und die beiden Elektroden (8) durch einen Spalt voneinander getrennt sind, wenn sich die Vorrichtung (1) im expandierten Zustand implantiert in das Blutgefäß (6) befindet.

9. Vorrichtung nach einem der Ansprüche 1 bis 8, **dadurch gekennzeichnet, dass** Elektroden (8) und/oder Paare von Elektroden (8) in Längsrichtung der Stentstruktur (2) betrachtet auf dem Umfang gegeneinander versetzt angeordnet sind.

10. Vorrichtung nach einem der Ansprüche 1 bis 9, **gekennzeichnet durch** Mittel zur Messung von elektrischen Widerständen.

11. Vorrichtung nach einem der Ansprüche 1 bis 10, **dadurch gekennzeichnet, dass** die Stentstruktur (2) auf der Innenseite eine Membran (11) aufweist.

## Claims

1. A device having a stent structure (2) which is intended for insertion into intracranial blood vessels (6) of the human or animal body, wherein the stent structure (2) has an expanded state in which it is capable of abutting the inner wall of the blood vessel (6) and a compressed state in which it is movable through the blood vessel (6) when the stent structure (2) is inside a microcatheter (4), wherein the stent structure (2) is connected to an insertion aid (3) and wherein the stent structure (2) is capable of automatically transitioning into the expanded state upon release from the microcatheter (4), wherein the stent structure (2) has electrical conductors (13) via which electrical pulses or high-frequency pulses can be applied to nerve fibres extending in the vessel wall of the blood vessel (6) in order to temporarily or permanently reduce the function of the nerve fibres and to prevent or treat vasospasm, and wherein the stent structure (2) has a spine (9) which runs from proximal to distal, from which struts (7) originate which have electrical conductors (13), which struts (7), in the expanded state, form the circumference of the stent structure (2), wherein pairs of electrical or high-frequency (HF) electrodes (8) connected to the electrical conductors (13) are arranged on the circumference of the stent structure (2) in such a manner that the electrodes (8) in the expanded state and implanted in the blood vessel (6) are spaced apart by a gap so that an applied current flow to the electrodes (8) acts across the gap on the inner wall of the blood vessel (6).

2. The device according to claim 1, **characterised in that** at least some struts (7) are composed of partial struts extending parallel to one another.

3. The device according to claim 1 or 2, **characterised in that** the electrical conductors (13) converge at the proximal end of the stent structure (2) and are connected to the insertion aid (3).

4. The device according to one of claims 1 to 3, **characterised in that** the electrical conductors (13) are electrically insulated from one another.

5. The device according to one of claims 1 to 4, **characterised in that** the electrodes (8) are provided with a radiographic marker (12).

6. The device according to one of claims 1 to 5, **characterised in that** the stent structure (2) is provided with a plurality of pairs of electrodes (8).

7. The device according to one of claims 1 to 6, **characterised in that** the gap between a pair of electrodes (8) is filled with an electrically insulating material.

8. The device according to one of claims 1 to 7, **characterised in that** the stent structure (2) comprises a plurality of substantially annular elements which are spaced apart in the longitudinal direction of the device (1), each comprising two electrical conductors (13) that belong to an electric circuit, with the two electrical conductors (13) each terminating in one electrode (8) and the two electrodes (8) being separated from each other by a gap when the device (1) is in the expanded state and implanted in the blood vessel (6).

9. The device according to one of claims 1 to 8, **characterised in that** electrodes (8) and/or pairs of electrodes (8), viewed in the longitudinal direction of the stent structure (2), are arranged offset relative to one another on the circumference.

10. The device according to one of claims 1 to 9, **characterised by** means for measuring electrical resistance.

11. The device according to one of claims 1 to 10, **characterised in that** the stent structure (2) has a membrane (11) on the inside.

## Revendications

1. Dispositif doté d'une structure de stent (2), qui est prévue pour être insérée dans des vaisseaux sanguins (6) intracrâniens du corps humain ou animal, la structure de stent (2) comportant un état déployé, dans lequel elle est apte à s'appliquer contre la paroi intérieure du vaisseau sanguin (6), et un état comprimé, dans lequel elle est déplaçable dans le vaisseau sanguin (6) à l'intérieur d'un microcathéter (4), la structure de stent (2) étant reliée à un guide d'insertion (3) et la structure de stent (2) étant apte à passer de manière autonome dans l'état déployé après sa libération hors du microcathéter (4), dans lequel la structure de stent (2) comporte des conducteurs électriques (13), par le biais desquels des impulsions électriques ou des impulsions haute fréquence peuvent être appliquées sur des fibres nerveuses s'étendant dans la paroi du vaisseau sanguin (6), pour diminuer temporairement ou durablement la fonction des fibres nerveuses et prévenir un vasospasme ou le traiter, et dans lequel la structure de stent (2) comporte une épine dorsale (9) s'étendant du côté proximal au côté distal, de laquelle partent des entretoises (7) comportant des conducteurs électriques (13), qui, dans l'état déployé, forment la circonférence de la structure de stent (2), des paires d'électrodes électriques ou à haute fréquence (8), qui sont raccordées aux conducteurs électriques (13), étant disposées sur la circonférence de la structure de stent (2) de telle manière que les électrodes (8) sont, dans l'état déployé et implanté dans le vaisseau sanguin (6), espacées les unes des autres par un interstice, de telle sorte qu'un flux de courant appliqué vers les électrodes (8) agit sur la paroi intérieure du vaisseau sanguin (6) par-delà l'interstice.

2. Dispositif selon la revendication 1, **caractérisé en ce qu'**au moins quelques entretoises (7) se composent d'entretoises partielles s'étendant parallèlement les unes aux autres.

3. Dispositif selon la revendication 1 ou 2, **caractérisé en ce que** les conducteurs électriques (13) convergent à l'extrémité proximale de la structure de stent (2) et sont reli0és au guide d'insertion (3).

4. Dispositif selon l'une des revendications 1 à 3, **caractérisé en ce que** les conducteurs électriques (13) sont isolés électriquement les uns des autres.

5. Dispositif selon l'une des revendications 1 à 4, **caractérisé en ce que** les électrodes (8) disposent d'un marquage radio-opaque (12).

6. Dispositif selon l'une des revendications 1 à 5, **caractérisé en ce que** la structure de stent (2) dispose de plusieurs paires d'électrodes (8).

7. Dispositif selon l'une des revendications 1 à 6, **caractérisé en ce que** l'interstice entre une paire d'électrodes (8) est rempli avec un matériau isolant électriquement.

8. Dispositif selon l'une des revendications 1 à 7, **caractérisé en ce que** la structure de stent (2) comporte plusieurs éléments sensiblement annulaires espacés dans la direction longitudinale du dispositif (1), qui comportent respectivement deux conducteurs électriques (13) appartenant à un circuit électrique, les deux conducteurs électriques (13) se terminant respectivement par une électrode (8) et les deux électrodes (8) étant séparées l'une de l'autre par un interstice quand le dispositif (1) se trouve dans l'état déployé implanté dans le vaisseau sanguin (6).

9. Dispositif selon l'une des revendications 1 à 8, **caractérisé en ce que** des électrodes (8) et/ou des paires d'électrodes (8) sont disposées décalées les unes par rapport aux autres sur la circonférence, lorsque l'on regarde dans la direction longitudinale de la structure de stent (2).

10. Dispositif selon l'une des revendications 1 à 9, **caractérisé par** des moyens de mesure de résistances électriques.

11. Dispositif selon l'une des revendications 1 à 10, **caractérisé en ce que** la structure de stent (2) comporte une membrane (11) sur son côté intérieur.
